(19) 

Europäisches
Patentamt
European
Patent Office
Office européen
des brevets

(11)  **EP 3 216 759 A1**

(12) **EUROPEAN PATENT APPLICATION**

published in accordance with Art. 153(4) EPC

(43) Date of publication:
**13.09.2017 Bulletin 2017/37**

(21) Application number: **15857005.1**

(22) Date of filing: **31.08.2015**

(51) Int Cl.:
*C01B 32/25* (2017.01)   *B82Y 30/00* (2011.01)
*B82Y 40/00* (2011.01)

(86) International application number:
**PCT/JP2015/074654**

(87) International publication number:
**WO 2016/072138 (12.05.2016 Gazette 2016/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **07.11.2014   JP 2014226656**

(71) Applicant: **Daicel Corporation
Osaka-shi, Osaka 530-0011 (JP)**

(72) Inventor: **YAMAKAWA, Akira
Himeji-shi
Hyogo 671-1283 (JP)**

(74) Representative: **Grünecker Patent- und
Rechtsanwälte
PartG mbB
Leopoldstraße 4
80802 München (DE)**

(54) **NANODIAMONDS HAVING ACID FUNCTIONAL GROUP AND METHOD FOR PRODUCING
SAME**

(57)   A nanodiamond according to the present invention has acidic functional groups, contains the acidic functional groups in a number density of 1 or more per square nanometer in the nanodiamond surface, and has a specific surface area of 150 $m^2$/g or more. Particles of the nanodiamond preferably have a D50 (median diameter) of 9 nm or less. The nanodiamond is preferably derived from a nanodiamond synthesized by a detonation technique (in particular, an air-cooling detonation technique).

## Description

Technical Field

The present invention relates to a nanodiamond having acidic functional groups, and a method for producing the same. This application claims priority to Japanese Patent Application No. 2014-226656, filed November 7, 2014 to Japan, the entire contents of which are incorporated herein by reference.

Background Art

Nanodiamond particles have characteristic properties such as high mechanical strengths, thermal conductivity, optical transparency, a low refractive index, excellent electrical insulation, a low dielectric constant, and a low friction coefficient and are thereby used typically as lubricants, surface modifiers, abrasives, and insulating materials for semiconductors (semiconductor devices) and circuit boards. In addition, applications of nanodiamonds to glass alternatives and to the electrical and electronic field, the energy field, and the biomedical field have been studied increasingly.

Such nanodiamond particles are produced by a static high-pressure process or a detonation technique. The nanodiamond particles, when produced by the detonation technique, are produced in the following manner. An explosive is detonated in a closed system to give detonation soot, the soot is purified by a chemical treatment, dispersed in water, and, in this state, pulverized using a disperser such as a bead mill or an ultrasonic homogenizer to give an aqueous dispersion, from which water is removed, and yields the nanodiamond as particles. The removal of water is performed by a technique such as ultracentrifugation, thickening-drying, freeze-drying, or a technique using a spray dryer.

Non Patent Literature (NPL) 1 reports as follows. A nanodiamond having a specific surface area of 386 $m^2$/g and having a surface acidic functional group (carboxy group) number of 0.81 per square nanometer was obtained by subjecting a nanodiamond synthesized by a detonation technique (detonation nanodiamond) to an acid treatment with a hydrogen fluoride-nitric acid mixture to remove metal impurities, and subsequently subjecting the resulting article to an oxidation treatment at 400°C in an air atmosphere to remove $sp^2$ carbon impurities. In addition, a nanodiamond having a specific surface area of 292 $m^2$/g and having a surface acidic functional group (carboxy group) number of 0.80 per square nanometer was obtained by subjecting a commercially available nanodiamond (having a specific surface area of 331 $m^2$/g and an acidic functional group (carboxy group) number of 0.15 per square nanometer) to an one-step acid-oxidation treatment with nitric acid at 240°C to 260°C and 100 atm.

Citation List

Non Patent Literature

NPL 1: Diamond & Related Materials 22 (2012) 113-117

Summary of Invention

Technical Problem

However, there has been no nanodiamond having acidic functional groups, where the nanodiamond offers sufficiently satisfactory dispersibility when dispersed in an ultrasmall size, in particular in a single-nano-size (single-digit-nano-size) in a dispersion medium.

The present invention has an object to provide a nanodiamond having acidic functional groups, and a method for industrially efficiently producing the nanodiamond, where the nanodiamond offers high dispersibility in an ultrasmall size, in particular in a single-nano-size. Solution to Problem

To achieve the object, the inventor of the present invention made investigations in detail on dispersibility of nanodiamonds in a dispersion medium, where the nanodiamonds are synthesized by a detonation technique (detonation nanodiamonds). As a result, the inventor found that a nanodiamond having a large specific surface area and having a very large number density of acidic functional groups per unit area is obtained by subjecting detonation nanodiamond soot to an acid treatment to give a nanodiamond (agglutinate) having an acidic functional group content at a specific level or more, and subjecting the nanodiamond (agglutinate) to an oxidation treatment under specific conditions; and that the resulting nanodiamond as above offers excellent dispersibility in a dispersion medium such as water. The present invention has been made on the basis of these findings and further investigations.

Specifically, the present invention provides a nanodiamond having acidic functional groups. The nanodiamond contains the acidic functional groups in a number density of 1 or more per square nanometer in the surface of the nanodiamond and has a specific surface area of 150 $m^2$/g or more.

Particles of the nanodiamond may have a D50 of typically 9 nm or less.

The nanodiamond is preferably derived from a nanodiamond synthesized by a detonation technique. The detonation technique may be an air-cooling detonation technique.

The nanodiamond is preferably a nanodiamond obtained by subjecting a nanodiamond having an acidic functional group content of 0.15 mmol/g or more to an oxidation treatment.

The present invention also provides a method for producing a nanodiamond, to produce the above-mentioned nanodiamond. The method includes the step A of subjecting a material nanodiamond to an oxidation treatment with at least one oxidizer selected from the group consisting of chromic acid, chromic anhydride, dichromic acid, permanganic acid, perchloric acid, salts of these acids, and hydrogen peroxide. The material nanodiamond is synthesized by a detonation technique and contains acidic functional groups in a content of 0.15 mmol/g or more.

In the production method, the oxidation treatment in the step A may be performed in the coexistence of sulfuric acid.

The oxidation treatment in the step A is preferably performed at a temperature of 130°C or higher.

The method may further include, before the step A, the step B of treating nanodiamond soot with a mineral acid to give the material nanodiamond having an acidic functional group content of 0.15 mmol/g or more, where the nanodiamond soot is formed by a detonation technique.

The method may further include, after the step A, the step C of subjecting a nanodiamond suspension resulting from the oxidation treatment to a deaggregation treatment.

Specifically, the present invention relates to the followings.

(1) A nanodiamond having acidic functional groups, wherein the nanodiamond contains the acidic functional groups in a number density of 1 or more per square nanometer in a surface of the nanodiamond, and wherein the nanodiamond has a specific surface area of 150 $m^2$/g or more.

(2) The nanodiamond according to (1), wherein particles of the nanodiamond have a D50 of 9 nm or less.

(3) The nanodiamond according to one of (1) and (2), wherein the nanodiamond is a detonation nanodiamond.

(4) The nanodiamond according to any one of (1) to (3), wherein the nanodiamond is is an air-cooled detonation nanodiamond.

(5) The nanodiamond according to any one of (1) to (4), wherein the nanodiamond is a nanodiamond obtained by subjecting a nanodiamond having an acidic functional group content of 0.15 mmol/g or more to an oxidation treatment.

(6) A method for producing a nanodiamond, to produce the nanodiamond according to any one of (1) to (5), wherein the method includes the step A of subjecting a material nanodiamond to an oxidation treatment with at least one oxidizer selected from the group consisting of chromic acid, chromic anhydride, dichromic acid, permanganic acid, perchloric acid, salts of these acids, and hydrogen peroxide, the material nanodiamond being synthesized by a detonation technique and having an acidic functional group content of 0.15 mmol/g or more.

(7) The method according to (6) for producing a nanodiamond, wherein the oxidation treatment in the step A is performed in water.

(8) The method according to one of (6) and (7) for producing a nanodiamond, wherein the oxidizer in the oxidation treatment is present in a concentration of 3 to 50 weight percent.

(9) The method according to any one of (6) to (8) for producing a nanodiamond, wherein the oxidizer in the oxidation treatment is used in an amount of 300 to 5000 parts by weight per 100 parts by weight of the nanodiamond.

(10) The method according to any one of (6) to (9) for producing a nanodiamond, wherein the oxidation treatment in the step A is performed in the coexistence of a mineral acid.

(11) The method according to (10) for producing a nanodiamond, wherein the mineral acid is sulfuric acid.

(12) The method according to (10) and (11) for producing a nanodiamond, wherein the mineral acid in the oxidation treatment is present in a concentration of 5 to 80 weight percent.

(13) The method according to any one of (6) to (12) for producing a nanodiamond, wherein the oxidation treatment in the step A is performed at a temperature of 130°C or higher.

(14) The method according to any one of (6) to (13) for producing a nanodiamond, the method further including, before the step A, the step B of treating nanodiamond soot with a mineral acid to give the material nanodiamond having an acidic functional group content of 0.15 mmol/g or more, where the nanodiamond soot is formed by a detonation technique.

(15) The method according to any one of (6) to (14) for producing a nanodiamond, the method further including, after the step A, the step C of subjecting a nanodiamond suspension resulting from the oxidation treatment to a deaggregation treatment.

Advantageous Effects of Invention

The nanodiamond having acidic functional groups according to the present invention has a large specific surface area, contains the acidic functional groups in a very high number density per unit area, and thereby offers excellent dispersibility in an ultrasmall size, in particular in a single-nano-size, in dispersion media such as water. The nanodiamond also has excellent dispersion stability.

The production method according to the present invention can industrially efficiently produce a nanodiamond having acidic functional groups and having such excellent properties as above.

Description of Embodiments

Nanodiamond Having Acidic Functional Groups

The nanodiamond according to the present invention has acidic functional groups in the surface of the nanodiamond. A non-limiting example of the acidic functional groups is a carboxy group. At least part of the acidic functional groups may form a salt. Non-limiting examples of the salt include alkali metal salts such as sodium salts and potassium salts; and alkaline earth metal salts such as calcium salts and magnesium salts. The nanodiamond has a number density (number per unit area) of the acidic functional groups of 1 or more per square nanometer. The number density is hereinafter also simply referred to as an "acidic functional group number". The nanodiamond according to the present invention has a specific surface area of 150 $m^2$/g or more. In the nanodiamond according to the present invention, at least part of the acidic functional groups may form a salt. As used herein, the term "acidic functional group number" refers to and means the total number of not only free acidic functional groups, but also acidic functional groups that are in the form of salts.

In the present invention, the acidic functional group number in the nanodiamond surface is preferably 1.1 or more per square nanometer, and more preferably 1.2 or more per square nanometer. The larger the acidic functional group number is, the better, but the upper limit of the acidic functional group number may typically be 2 per square nanometer. The specific surface area is preferably 200 $m^2$/g or more, more preferably 250 $m^2$/g or more, and particularly preferably 280 $m^2$/g or more. The upper limit of the specific surface area is typically 500 $m^2$/g and may be 400 $m^2$/g.

Assume that a nanodiamond has an acidic functional group number in the nanodiamond surface and a specific surface area within the ranges. This nanodiamond offers excellent dispersibility in dispersion media such as water, even having an ultrasmall size, in particular having a single-nano-size (having a D50 (median diameter) of 9 nm or less). In addition, this nanodiamond offers higher functionalities owing to the acidic functional groups. In contrast, assume that a nanodiamond has an acidic functional group number and/or a specific surface area out of the range(s). This nanodiamond offers lower dispersibility in dispersion media such as water.

In the nanodiamond according to the present invention, particles of the nanodiamond preferably have a smaller D50 (median diameter) from the point of higher performance in use in various applications. The nanodiamond particles may have a D 50 of typically 200 nm or less, preferably 100 nm or less, more preferably 50 nm or less, furthermore preferably 9 nm or less, and particularly preferably 7 nm or less. The lower limit of the D50 is typically 3.5 nm and may be about 4 nm.

The nanodiamond according to the present invention is preferably a nanodiamond derived from a nanodiamond synthesized by a detonation technique (in particular, an air-cooling detonation technique). This is preferred in terms of excellent dispersibility (in particular, single-nano-sized dispersibility). The nanodiamond according to the present invention is preferably a nanodiamond obtained by subjecting a material nanodiamond to an oxidation treatment, where the material nanodiamond has an acidic functional group content of 0.15 mmol/g or more (more preferably 0.2 mmol/g or more, furthermore preferably 0.3 mmol/g or more, and particularly preferably 0.4 mmol/g or more), where the "acidic functional group content" refers to the content of acidic functional groups. This is preferred in terms of excellent dispersibility (in particular, single-nano-size dispersibility). As used herein, the term "acidic functional group content" refers to the total content of not only free acidic functional groups, but also acidic functional groups that are in the form of salts.

Production of Nanodiamond Having Acidic Functional Groups

The nanodiamond having acidic functional groups according to the present invention can be produced from artificial nanodiamond particles. The artificial nanodiamond particles can be produced typically from an element mineral of carbon (such as graphite) as a raw material typically via a process or technique such as a detonation technique, a flux growth, a static high-pressure process, or a high-temperature and high-pressure process. The nanodiamond according to the present invention is preferably one derived from a nanodiamond formed by a detonation technique (in particular, a detonation technique using an oxygen-deficient explosive). This is preferred for obtaining nanodiamond particles having an extremely small median diameter of primary particles.

The detonation technique is a technique of detonating an explosive and thereby applying a dynamic impact to an element mineral of carbon, to convert the carbon element mineral directly into particles having a diamond structure. Examples of the explosive for use herein include, but are not limited to, cyclotrimethylenetrinitroamine (RDX), cyclotetramethylenetetranitramine (HMX), trinitrotoluene (TNT), trinitrophenylmethylnitroamine, pentaerythritol tetranitrate, tetranitromethane, and mixtures of them (such as TNT/HMX and TNT/RDX mixtures).

Such detonation techniques are classified by the heat removing process into a water-cooling detonation technique and an air-cooling detonation technique. As compared with the water-cooling detonation technique, the air-cooling detonation technique gives nanodiamonds which have surface functional groups in a larger amount, have higher particle surface hydrophilicity, and, in addition, have a smaller particle size distribution in an aqueous dispersion because of having a smaller primary particle diameter. Accordingly, the nanodiamond according to the present invention is particularly preferably one derived from an air-cooled detonation nanodiamond (nanodiamond synthesized by the air-cooling detonation technique).

The nanodiamond particles (nanodiamond soot) obtained by the above method tend to be contaminated with oxides of metals such as Fe, Co, and Ni, which metals are contained typically in the production equipment, where non-limiting examples of the metal oxides include $Fe_2O_3$, $Fe_3O_4$, $Co_2O_3$, $Co_3O_4$, NiO, and $Ni_2O_3$. Accordingly, the nanodiamond particles (nanodiamond soot) obtained by the method are preferably subjected to an acid treatment with a strong acid, to dissolve and remove the oxides of metals (i.e., metal oxides).

The strong acid for use in the acid treatment is preferably selected from mineral acids such as hydrochloric acid, hydrofluoric acid, sulfuric acid, nitric acid, and aqua regia. Each of them may be used alone or in combination.

The acid treatment is generally performed in water. The acid treatment may employ the strong acid (such as a mineral acid) in a concentration of typically 1 to 50 weight percent, preferably 3 to 30 weight percent, and more preferably 5 to 20 weight percent. The acid treatment may be performed for a time of typically 0.1 to 24 hours, preferably 0.2 to 10 hours, and more preferably 0.3 to 5 hours. The acid treatment may be performed at a temperature of typically 70°C to 150°C, preferably 90°C to 130°C, and more preferably 100°C to 125°C. The acid treatment may be performed under any pressure, such as under reduced pressure, at normal atmospheric pressure, or under pressure (under a load), but is preferably performed at normal atmospheric pressure. This is preferred typically in terms of operability and facilities.

The nanodiamond soot contains not only the metal components, but also graphite. To remove the graphite, the nanodiamond soot (preferably, one obtained by subjecting the nanodiamond soot to the acid treatment) is preferably subjected to an oxidation treatment.

To give a nanodiamond having acidic functional groups and having an acidic functional group number of 1 or more per square nanometer in the nanodiamond surface, the nanodiamond (including graphite) to be subjected to the oxidation treatment preferably contains acidic functional groups in a content of 0.15 mmol/g or more. Namely, a nanodiamond (including graphite) having an acidic functional group content of 0.15 mmol/g or more is preferably selected so as to be subjected to the oxidation treatment. The acidic functional group content is more preferably 0.2 mmol/g or more, furthermore preferably 0.3 mmol/g or more, and particularly preferably 0.4 mmol/g or more. The higher the acidic functional group content is, the better, but the upper limit of the content is typically 2 mmol/g and may be about 1 mmol/g.

The acidic functional group content of the nanodiamond to be subjected to the oxidation treatment can be adjusted typically by detonation conditions such as the type of the explosive, and the gas atmosphere in a furnace.

Non-limiting examples of the oxidizer for use in the oxidation treatment include concentrated nitric acid, fuming nitric acid, and fuming sulfuric acid; chromic acid, chromic anhydride, dichromic acid, permanganic acid, perchloric acid, and salts of these acids; and hydrogen peroxide. Each of them may be used alone or in combination. Among them, the oxidizer for use herein is preferably at least one selected from the group consisting of chromic acid, chromic anhydride, dichromic acid, permanganic acid, perchloric acid, salts of these acids, and hydrogen peroxide.

The oxidation treatment is generally performed in a solvent. The solvent is preferably water. The oxidation treatment may employ the oxidizer in a concentration of typically 3 to 50 weight percent, preferably 6 to 30 weight percent, and more preferably 8 to 20 weight percent. The oxidation treatment may employ the oxidizer in an amount of typically 300 to 5000 parts by weight, preferably 500 to 3000 parts by weight, and more preferably 800 to 2000 parts by weight, per 100 parts by weight of the nanodiamond.

The oxidation treatment is preferably performed in the coexistence of a mineral acid in view of efficiency in graphite removal. Non-limiting examples of the mineral acid are as with those exemplified above. The mineral acid is preferably sulfuric acid. The mineral acid (such as sulfuric acid), when used in the oxidation treatment, may be present in a concentration of typically 5 to 80 weight percent, preferably 10 to 70 weight percent, and more preferably 20 to 60 weight percent.

The oxidation treatment may be performed for a treatment time of typically one hour or longer (e.g., 1 to 24 hours), preferably 2 hours or longer (e.g., 2 to 15 hours), and more preferably 3 hours or longer (e.g., 3 to 10 hours). This is preferred from the viewpoint of allowing the nanodiamond to have a larger acidic functional group number in the surface and to have a larger specific surface area. The oxidation treatment may be performed for a treatment temperature of typically 100°C or higher (e.g., 100°C to 200°C), preferably 120°C or more (e.g., 120°C to 180°C), more preferably 130°C or higher (e.g., 130°C to 160°C), and particularly preferably 135°C or higher (e.g., 135°C to 150°C). This is preferred from the viewpoint of allowing the nanodiamond to have a larger acidic functional group number in the surface and to have a larger specific surface area. The oxidation treatment may be performed under any pressure, such as under reduced pressure, at normal atmospheric pressure, or under pressure (under a load), but is preferably performed at normal atmospheric pressure typically in terms of operability and facilities. The oxidation treatment, even when performed under pressure, is preferably performed at a pressure of 5 MPa or less. Accordingly, the pressure is preferably 0.1 to 5 MPa, more preferably 0.1 to 1 MPa, and furthermore preferably 0.1 to 0.5 MPa.

Nanodiamond particles obtained by subjecting the nanodiamond soot to the acid treatment are generally present as aggregates (agglutinates) in which a graphite layer is deposited on nanodiamond primary particles, and the graphite layer entangles two or more primary particles to form a so-called aggregate structure, and the aggregate structure offers a firmer aggregation state as compared with a structure formed by van der Waals cohesion. Nanodiamond particles, which are obtained by subjecting the nanodiamond soot as intact or after the acid treatment to the oxidation treatment, are generally present as aggregates in which nanodiamond primary particles undergo interparticle cohesion (van der Waals cohesion). In the description, the agglutinates and the aggregates formed by van der Waals cohesion are also collectively referred to as "nanodiamond aggregates". The nanodiamond aggregates have a D50 (median diameter) of generally 20 nm or more and usually from 100 nm to 10 $\mu$m.

After the oxidation treatment, washing with water (such as pure water or ion-exchanged water) is performed to give nanodiamond particles (aggregates).

The nanodiamond surface acidic functional groups (such as carboxy groups) can be converted into salts (such as carboxylates) by treating the nanodiamond after the oxidation treatment with an alkaline solution (such as a sodium hydroxide aqueous solution). The alkaline treatment may be performed at an alkali concentration of typically 1 to 50 weight percent, preferably 3 to 30 weight percent, and more preferably 5 to 20 weight percent. The alkaline treatment may be performed at a temperature of typically 70°C to 150°C, preferably 90°C to 130°C, and more preferably 100°C to 125°C, for a time of typically 0.1 to 24 hours, preferably 0.2 to 10 hours, and more preferably 0.3 to 5 hours. The nanodiamond can have free surface acidic functional groups again by treating the nanodiamond after the alkaline treatment with an acid (such as hydrochloric acid). The acid treatment may be performed at room temperature or with heating.

Repeated water washing can remove electrolytes (such as NaCl), which are impurities, from the nanodiamond after the oxidation treatment, one resulting from the alkaline treatment of the nanodiamond after the oxidation treatment, and one resulting from the acid treatment of the nanodiamond after the alkaline treatment. Removal of such electrolytes allows the nanodiamond to disperse more satisfactorily (finely) and more stably.

Assume that the nanodiamond particles (aggregates) obtained in the above manner (which may be further subjected to a classification treatment as needed) are dispersed in a dispersion medium to give a suspension, and the suspension is subjected to a deaggregation treatment. This gives ultrasmall-sized, in particular, single-nano-sized (single-digit-nano-sized) nanodiamond particles. The deaggregation treatment is also referred to as a "dispersing treatment". In the present invention, the term "deaggregation" is used in such a broad meaning as to include not only deaggregation of nanodiamond aggregates, but also deagglutination of agglutinated nanodiamond.

Non-limiting examples of the dispersion medium include water; polar organic solvents exemplified by alcohols such as methanol, ethanol, and ethylene glycol, ketones such as acetone, and lactams or amides such as N-methylpyrrolidone; and mixtures of these solvents. Among them, dispersion media containing water (such as one containing 50 weight percent or more of water) are preferred, and water is particularly preferred.

The dispersing treatment may be performed using a disperser. Non-limiting examples of the disperser include high-shearing mixers, high-shear mixers, homomixers, ball mills, bead mills, high-pressure homogenizers, ultrasonic homogenizers, and colloid mills. Among them, the dispersing is preferably performed using a bead mill and/or an ultrasonic homogenizer in terms of efficiency.

The suspension of nanodiamond, when subjected to the dispersing treatment, is preferably adjusted to have a pH of 8 or more (e.g., 8 to 12), preferably 9 or more (e.g., 9 to 11), and more preferably 9.5 to 10.5 during the dispersing treatment. This is preferred for allowing the finally-obtained nanodiamond particles to disperse more satisfactorily and more stably. After the dispersing treatment, the product may further be subjected to a classification treatment as needed.

Removal of water from the nanodiamond dispersion (suspension) obtained in the above manner can give a nanodiamond powder. The removal may be performed by a known technique such as ultracentrifugation, thickening-drying, freeze-drying, or a technique using a spray dryer.

Examples

The present invention will be illustrated in further detail with reference to several examples below. It should be noted, however, that the examples are by no means intended to limit the scope of the present invention. Percentages are by weight.

Properties of suspensions, dispersions, and nanodiamonds were measured by methods below.

pH

Hydrogen ion concentrations (pHs) of the suspensions and dispersions were measured using D-51 (trade name) supplied by HORIBA, Ltd.

Solids Concentration

Solids contents of the suspensions and dispersions were calculated each by evaporating the solvent (water) of a sample suspension or dispersion on a sand bath heated up to 250°C, and determining the amount of solids.

D50 (Median Diameter)

D50s of nanodiamond particles were determined using Zetasizer Nano ZS (trade name) supplied by Spectris Co., Ltd. via dynamic light scattering (noncontact backscatter technique).

Specific Surface Area

Specific surface Areas of the nanodiamonds were measured using BELSORP-max supplied by Bel Japan, Inc.

Acidic Functional Group Number and Acidic Functional Group Content

A sample nanodiamond powder (0.25 g) was combined with 0.05 N sodium hydroxide aqueous solution (25 g), followed by stirring using a stirrer for 24 hours to give a suspension. The suspension was subjected to centrifugal separation (at 3000 rpm for 20 min). The content (mmol/g) of nanodiamond surface acidic functional groups was determined by subjecting 20 g of a supernatant, from which nanodiamond had been removed, to neutralization titration with 0.1 N hydrochloric acid. On the basis of the acidic functional group content (mmol/g) and the specific surface area (m$^2$/g), the acidic functional group number was determined according to expression as follows:

$$\text{Acidic functional group number (per square nanometer)} = \{(\text{Acidic functional group content (mmol/g)}) \times 6 \times 10^{23} \times 10^{-3}\}/\{(\text{Specific surface area (m}^2/\text{g)}) \times 10^9 \times 10^9\}$$

Example 1

An air-cooled detonation nanodiamond soot (ALIT Inc., Czech) having a nanodiamond primary particle diameter of 4 to 6 nm was weighed (220 g), combined with 10.7 L of 10% hydrochloric acid, and heated under reflux for one hour (acid treatment). After cooling, water washing was performed via decantation repeatedly until the pH of a precipitate mixture reached 2.5, followed by removal of supernatants as much as possible. Nanodiamonds (including graphite) contained in the precipitate mixture had an acidic functional group content of 0.43 mmol/g, where the content was measured using a powder obtained by drying part of the precipitate mixture.

Next, 1625 g (solids content: 125 g) of the precipitate mixture were combined with 4725 g of 98% sulfuric acid and 50 g of ultrapure water, where the procedure was performed at 40°C or lower. This was combined with 3050 g (chromic acid: 1500 g) of a chromic acid aqueous solution, heated under reflux (internal temperature: 141°C) for 5 hours, cooled, and yielded 9450 g of a slurry (oxidation treatment).

Water washing was performed via decantation repeatedly until the supernatants became colorless, followed by removal of the supernatants as much as possible. The resulting nanodiamond (aggregates) obtained by the oxidation treatment had a D50 of 4.3 nm, where the D50 was an average of primary particle diameters determined by XRD.

The above-obtained precipitate mixture was combined with 1 L of 10% sodium hydroxide aqueous solution, followed by a heat treatment under reflux for one hour. After cooling, a supernatant was removed by decantation, and 20% hydrochloric acid was added to adjust the pH to 2.5, followed by water washing via centrifugal sedimentation. The final-centrifuged precipitates were diluted with ultrapure water to be adjusted to have a solids concentration of 8%, was adjusted to have a pH of 10 with sodium hydroxide, and yielded a slurry before dispersion.

The slurry before dispersion was subjected to dispersion using a bead mill. The bead mill used herein was ULTRA APEX MILL UAM-015 supplied by KOTOBUKI INDUSTRIES CO., LTD. After charging zirconia beads having a diameter of 0.03 mm, which are deaggregation media, into a milling chamber up to 60% of the chamber volume, 300 mL of the slurry before dispersion were circulated at a flow rate of 10 L/hr, and subjected to deaggregation for 90 minutes at a set peripheral speed of 10 m/s. The resulting mixture after deaggregation was recovered, from which coarse particles were removed by a classification operation via centrifugal separation, and yielded a nanodiamond dispersion having a D50 in terms of particle size distribution of 5.4 nm.

The nanodiamond dispersion was placed into a dialysis membrane. Milli-Q (ultrapure water) was used as an external fluid in an amount 100 times as much as the amount of the internal fluid in the dialysis membrane. The external fluid was replaced every dialysis for 6 hours or longer, and the dialysis was completed at the time point when the pH of the external fluid became 7. The internal fluid was recovered, dried using an evaporator and a vacuum dryer (50°C, overnight), and yielded a nanodiamond powder. The nanodiamond powder had a specific surface area of 290 $m^2/g$ and an acidic functional group number of 1.25 per square nanometer.

Example 2

An air-cooled detonation nanodiamond soot (ALIT Inc., Czech; produced in a production lot different from that of the nanodiamond soot used in Example 1) having a nanodiamond primary particle diameter of 4 to 6 nm was weighed (220 g), combined with 10.7 L of 10% hydrochloric acid, followed by heating under reflux for one hour (acid treatment). After cooling, water washing via decantation was performed repeatedly until the pH of the resulting precipitate mixture reached 2.5, followed by removal of supernatants as much as possible. The nanodiamond (including graphite) contained in the precipitate mixture had an acidic functional group content of 0.27 mmol/g, where the content was measured using a powder obtained by drying part of the precipitate mixture.

This precipitate mixture was subjected to an oxidation treatment, a pH adjustment, and a deaggregation (dispersing treatment) by a procedure similar to that in Example 1, and yielded a nanodiamond dispersion having a particle size distribution D50 of 18.8 nm.

The nanodiamond dispersion was subjected to dialysis and dried by a procedure similar to that in Example 1, and yielded a nanodiamond powder. This nanodiamond powder had a specific surface area of 227 $m^2/g$ and an acidic functional group number of 1.02 per square nanometer.

Comparative Example 1

A nanodiamond dispersion was obtained by a procedure similar to that in Example 1, except for using a water-cooled detonation nanodiamond soot (produced in Ukraine) having a nanodiamond primary particle diameter of 5 to 7 nm, instead of the air-cooled detonation nanodiamond soot (ALIT Inc., Czech) having a nanodiamond primary particle diameter of 4 to 6 nm used in Example 1. The nanodiamond in the prepared nanodiamond dispersion had a particle size distribution D50 of 51.7 nm. The nanodiamond dispersion was subjected to dialysis and was dried by a procedure similar to that in Example 1 to give a nanodiamond powder, and the nanodiamond powder was found to have a specific surface area of 228 $m^2/g$ and an acidic functional group number of 0.5 per square nanometer.

Industrial Applicability

The nanodiamond having acidic functional groups according to the present invention offers excellent dispersibility and still has excellent dispersion stability in dispersion media such as water even in an ultrasmall size, in particular, in a single-nano-size (in a single-digit-nano size). The production method according to the present invention can industrially efficiently produce a nanodiamond having acidic functional groups and having such excellent properties as above.

**Claims**

1. A nanodiamond having acidic functional groups,
   the nanodiamond comprising the acidic functional groups in a number density of 1 or more per square nanometer in a surface of the nanodiamond,
   the nanodiamond having a specific surface area of 150 $m^2/g$ or more.

2. The nanodiamond according to claim 1,
   wherein particles of the nanodiamond have a D50 of 9 nm or less.

3. The nanodiamond according to one of claims 1 and 2,
   wherein the nanodiamond is a detonation nanodiamond.

4. The nanodiamond according to any one of claims 1 to 3,
   wherein the nanodiamond is an air-cooled detonation nanodiamond.

5. The nanodiamond according to any one of claims 1 to 4,
   wherein the nanodiamond is a nanodiamond obtained by subjecting a nanodiamond having an acidic functional group content of 0.15 mmol/g or more to an oxidation treatment.

6. A method for producing a nanodiamond, to produce the nanodiamond according to any one of claims 1 to 5, the method comprising the step of

   A) subjecting a material nanodiamond to an oxidation treatment with at least one oxidizer selected from the group consisting of chromic acid, chromic anhydride, dichromic acid, permanganic acid, perchloric acid, salts of these acids, and hydrogen peroxide, the material nanodiamond being synthesized by a detonation technique and having an acidic functional group content of 0.15 mmol/g or more.

7. The method according to claim 6 for producing a nanodiamond,
   wherein the oxidation treatment in the step A) is performed in coexistence of sulfuric acid.

8. The method according to one of claims 6 and 7 for producing a nanodiamond,
   wherein the oxidation treatment in the step A) is performed at a temperature of 130°C or higher.

9. The method according to any one of claims 6 to 8 for producing a nanodiamond, the method further comprising, before the step A), the step of

   B) treating nanodiamond soot with a mineral acid to give the material nanodiamond having an acidic functional group content of 0.15 mmol/g or more, the nanodiamond soot being formed by a detonation technique.

10. The method according to any one of claims 6 to 9 for producing a nanodiamond, the method further comprising, after the step A, the step of

    C) subjecting a nanodiamond suspension resulting from the oxidation treatment to a deaggregation treatment.

<table>
<tr><td colspan="2"><strong>INTERNATIONAL SEARCH REPORT</strong></td><td colspan="2">International application No.<br>PCT/JP2015/074654</td></tr>
</table>

**A. CLASSIFICATION OF SUBJECT MATTER**
*C01B31/06*(2006.01)i, *B82Y30/00*(2011.01)i, *B82Y40/00*(2011.01)i

According to International Patent Classification (IPC) or to both national classification and IPC

**B. FIELDS SEARCHED**

Minimum documentation searched (classification system followed by classification symbols)
C01B31/00-31/36, B82Y30/00, B82Y40/00, B01J13/00

Documentation searched other than minimum documentation to the extent that such documents are included in the fields searched
Jitsuyo Shinan Koho        1922-1996   Jitsuyo Shinan Toroku Koho   1996-2015
Kokai Jitsuyo Shinan Koho  1971-2015   Toroku Jitsuyo Shinan Koho   1994-2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2013-56805 A (Vision Development Co., Ltd.), 28 March 2013 (28.03.2013), paragraphs [0035] to [0037], [0040], [0045] to [0048], [0056] to [0057] (Family: none) | 1-10 |
| A | JP 2012-135718 A (Vision Development Co., Ltd.), 19 July 2012 (19.07.2012), paragraphs [0041] to [0044], [0049] to [0050] (Family: none) | 1-10 |
| A | JP 2014-144903 A (Vision Development Co., Ltd.), 14 August 2014 (14.08.2014), entire text (Family: none) | 1-10 |

☒ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 October 2015 (09.10.15) | 20 October 2015 (20.10.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.

PCT/JP2015/074654

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2013-10991 A (Tomei Diamond Co., Ltd.),<br>17 January 2013 (17.01.2013),<br>entire text<br>(Family: none) | 1-10 |
| A | WO 2009/060613 A1 (Nippon Kayaku Co., Ltd.),<br>14 May 2009 (14.05.2009),<br>entire text<br>& JP 5364588 B | 1-10 |

Form PCT/ISA/210 (continuation of second sheet) (July 2009)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014226656 A

**Non-patent literature cited in the description**

- *Diamond & Related Materials,* 2012, vol. 22, 113-117